# EUROPÄISCHE PATENTSCHRIFT

(11) **EP 4 140 412 B1**
(45) Veröffentlichungstag und Bekanntmachung des Hinweises auf die Patenterteilung: **15.05.2024**
(21) Anmeldenummer: 21193713.1
(22) Anmeldetag: 30.08.2021
(51) Int. Cl.: A61B 6/03, A61B 6/51, A61B 6/00

(54) **VERFAHREN ZUR DIGITALEN VOLUMEN TOMOGRAPHIE AUFNAHME EINES KLEINEN VOLUMENS**
METHOD FOR DIGITAL VOLUME TOMOGRAPHY OF A SMALL VOLUME
PROCÉDÉ D'ENREGISTREMENT D'UN PETIT VOLUME PAR TOMOGRAPHIE VOLUMÉTRIQUE NUMÉRIQUE

(43) Veröffentlichungstag der Anmeldung: 01.03.2023
(73) Patentinhaber: Dentsply Sirona Inc., York, PA 17401 (US); Sirona Dental Systems GmbH, 64625 Bensheim (DE)
(72) Erfinder: BLECHER, Wolf, 64625 Bensheim (DE); ELVERS, Michael, 64625 Bensheim (DE)
(74) Vertreter: Özer, Alpdeniz

(56) Entgegenhaltungen:
- EP-A1- 3 461 417
- EP-A2- 2 613 702
- JP-A- 2009 136 665
- US-A1- 2004 247 069
- KAASALAINEN TOUKO ET AL: "Dental cone beam CT: An updated review", PHYSICA MEDICA, Bd. 88, 17. Juli 2021 (2021-07-17), Seiten 193-217, XP055889891, IT ISSN: 1120-1797, DOI: 10.1016/j.ejmp.2021.07.007
- GOMES JULIANA ET AL: "An investigation of low-dose 3D scout scans for computed tomography", PROGRESS IN BIOMEDICAL OPTICS AND IMAGING, SPIE - INTERNATIONAL SOCIETY FOR OPTICAL ENGINEERING, BELLINGHAM, WA, US, Bd. 10132, 3. März 2017 (2017-03-03), Seiten 101322M-101322M, XP060087638, ISSN: 1605-7422, DOI: 10.1117/12.2255514 ISBN: 978-1-5106-0027-0

## Beschreibung

### TECHNISCHES GEBIET DER ERFINDUNG

Die vorliegende Erfindung bezieht sich auf ein Verfahren zur digitalen Volumen Tomographie (DVT) Aufnahme im dentalen Bereich.

### HINTERGRUND DER ERFINDUNG

Die Wahl eines kleinen Volumens für eine 3D Aufnahme spielt insbesondere in der Endodontie eine große Rolle. Durch die 3D Aufnahmen erhält der Behandler wichtige Informationen zur Lage und zum Verlauf der Wurzelkanäle, die für einen Erfolg der Behandlung entscheidend sein können. Durch ein kleines Volumen (z.B. gerade ausreichend für einen einzelnen Zahn) wird die Dosisbelastung für den Patienten minimiert, ohne dem Behandler den zusätzlichen Erkenntnisgewinn im Vergleich zu einer 2D Aufnahme zu verwehren. Die Schwierigkeit bei einer auf einen einzelnen Zahn beschränkten Aufnahme liegt zum einen in der exakten Positionierung des Volumens, zum anderen in der genauen Ausführung der Aufnahme mit dem kleinen Volumen. Insbesondere bei DVT Geräten, die *keine reine Kreisbahn* fahren und dadurch z.B. eine größere Schulterfreiheit bei kleinerem Platzbedarf der Geräte bieten, werden einzelne verfügbare Drehzentren kalibriert, um durch die Kenntnis des genauen Laufes des Gerätes eine gute Rekonstruktion der aufgenommenen Daten zu ermöglichen. Hier ist es derzeit nicht möglich ein Volumen einer kleinen Größe beliebig im maximal möglichen Field-Of-View zu platzieren und gleichzeitig eine gute Rekonstruktion zu ermöglichen. Im allgemeinen kann eine freie Platzierung eines kleinen Volumens über eine Verschiebung des Rotationszentrums und der Fahrt *einer Kreisbahn* um den gewählten Punkt realisiert werden Die Auswahl eines kleinen Drehzentrums (z.B. 2x2cm oder 4x4cm) wird bei aktuell bekannten Röntgengeräten über z.B. eine Panorama Scout Aufnahme oder eine Live-View Video-Positionierung realisiert.

EP 3 461 417 A1 offenbart ein Verfahren zur digitalen Volumen Tomographie (DVT) Aufnahme im dentalen Bereich, wobei auf einer 3D Scout-Aufnahme ein Bereich ausgewählt und mit einer weiteren 3D Messung aufgenommen wird.

### OFFENBARUNG DER ERFINDUNG

Die vorliegende Erfindungsmeldung hat das Ziel ein Verfahren bereitzustellen mit dem man bei einer Anzahl an verfügbaren kalibrierten Drehzentren jeweils mit oder ohne Fahrt einer Kreisbahn, ein kleines Volumen an beliebiger Position im maximal möglichen Field of View (FoV) des DVT Röntgengerätes platzieren, aufnehmen und rekonstruieren kann. Dieses Ziel wird durch die Verfahren nach einem der Ansprüche 1 bis 3 erreicht. Die Gegenstände der abhängigen Ansprüche beziehen sich auf Weiterentwicklungen.

Das erfindungsgemäße Verfahren dient zur digitalen Volumen Tomographie (DVT) Aufnahme im dentalen Bereich mit einem Röntgengerät. Es umfasst die folgenden Schritte: (a) Ein Patient wird im Röntgengerät positioniert; (b) Es wird eine 3D Scoutaufnahme mit reduzierter Dosis und mit einer von mehreren vordefinierten Volumengrößen und einem vordefinierten Drehzentrun des Röntgengeräts erstellt; (c) Auf der 3D Scoutaufnahme markiert ein Behandler einen Zahn, für den er hochauflösende Informationen haben möchte, mit einer umhüllenden Geometrie; (d) Eine Software bestimmt anhand einer Position der umhüllenden Geometrie die verfügbaren Drehzentren des Röntgengeräts, bei denen diese umhüllende Geometrie abgebildet werden kann, und errechnet anhand von Referenzpunkten der umhüllenden Geometrie Abbildungspositionen auf einem Röntgendetektor des Röntgengeräts für jedes der verfügbaren Drehzentren; (e) Anhand der Abbildungspositionen wird ein Drehzentrum aus der Menge der verfügbaren Drehzentren ausgewählt, und zu diesem Drehzentrum werden Blendenpositionen für eine hochauflösende Aufnahme und zu bestrahlende Detektorbereiche der hochauflösenden Aufnahme ermittelt; (f) Die zuvor ermittelnden Blendenpositionen und die zu bestrahlenden Detektorbereiche werden an das Röntgengerät übermittelt und nun die hochauflösende Aufnahme mit dem aus der Menge der verfügbaren Drehzentren ausgewählten Drehzentrum und den übermittelten Blendenpositionen durchgeführt; (g) Die hochauflösende Aufnahme wird rekonstruiert und in der 3D Scoutaufnahme eine entsprechende neu aufgenommene hochauflösende Information eingeblendet; (h) Der Behandler erhält zur Befundung und weiteren Verwendung die 3D Scoutaufnahme dargestellt, bei der die zuvor markierte umhüllende Geometrie durch die hochauflösende Information überlagert ist.

Ein wesentlicher vorteilhafter Effekt der vorliegenden Erfindung ist, dass mit dem hier vorgeschlagenen Verfahren es erstmals möglich wird, bei DVT-Geräten mit vordefinierten kalibrierten Drehzentren ein Volumen einer nahezu beliebigen Größe an einem beliebigen Ort innerhalb des maximal möglichen FoV zu positionieren, aufzunehmen und zu rekonstruieren.

Ein weiterer wesentlicher vorteilhafter Effekt der vorliegenden Erfindung ist dass durch die Vorberechnung und Kommandierung der unterschiedlichen Blendenpositionen während der Aufnahme anhand des ausgewählten Drehzentrums der gewünschte Abbildungsbereich genau abgebildet werden kann.

In einer Variante des erfindungsgemäßen Verfahrens werden die Schritte (b) und (c) durch die folgenden Schritte ersetzt: (b1') Der Behandler markiert den Zahn, für den er hochauflösende Informationen haben möchte anhand eines Zahnschemas; (b2') Das Röntgengerät bestimmt automatisiert das optimale kalibrierte Drehzentrum für die 3D Scoutaufnahme, dass diesen markierten Zahn sicher abbildet; (b3') Es wird eine 3D Scoutaufnahme mit reduzierter Dosis und mit einer der vordefinierten Volumengrößen und dem optimalen verfügbaren kalibrierten Drehzentrum aus dem vorangegangenen Schritt erstellt; (c') mittels einer Software wird der markierte Zahn auf der 3D Scoutaufnahme gefunden und eine, die den markierten Zahn umhüllende Geometrie automatisch bestimmt.

In einer weiteren Variante des erfindungsgemäßen Verfahrens werden die Schritte (b) und (c) durch die folgenden Schritte ersetzt: (b") Der Behandler bestimmt eine Region auf einer Bedienoberfläche für die er hochauflösende Informationen haben möchte; (c") Eine Software bestimmt anhand der gewählten Region und der Position der geschlossenen Schläfenstützen oder eines anderen Hilfsmittels eine umhüllende Geometrie. In dieser Alternative wird auf die 3D Scoutaufnahme verzichtet. Daher fällt der Schritt (h) weg. Und der Schritt (g) wird durch den folgenden Schritt ersetzt: (g') Die hochauflösende Aufnahme wird rekonstruiert und dem Behandler zur Befundung dargestellt.

In weiteren bevorzugten Varianten der erfindungsgemäßen Verfahren wird im Schritt (e) anhand der Abbildungspositionen das Drehzentrum ausgewählt, dass die *vorteilhafteste* Abbildungsposition auf dem Röntgendetektor bietet. Bei der Ermittlung der vorteilhaftesten Abbildungsposition werden Einflüsse wie, z.B. die Abbildungsverzerrung durch den Anodenwinkel berücksichtigt. Alternativ oder zusätzlich wird bei der Ermittlung der vorteilhaftesten Abbildungsposition eines oder mehrere der Kriterien wie Patient-Detektor-Abstand, Minimierung der Effektivdosis, Minimierung der Blendenbewegung, Minimierung der Anzahl der Blendenpositionen, oder Minimierung der Größe der zu bestrahlenden Detektorbereiche berücksichtigt.

### KURZBESCHREIBUNG DER ZEICHNUNGEN

In der nachfolgenden Beschreibung wird die vorliegende Erfindung anhand von beispielhaften Ausführungsformen und unter Bezugnahme auf die Zeichnungen näher erläutert, wobei
Abb.1 - zeigt ein DVT System zur digitalen Volumen Tomographie (DVT) Aufnahme gemäß einer Ausführungsform,
Abb.2a - zeigt die Bedienungseinheit des DVT Systems aus Abb. 1, wobei der Behandler mit einem vordefinierten Drehzentrum und einer vordefinierten Volumengröße eine 3D Scoutaufnahme ausführt;
Abb.2b - zeigt die Bedienungseinheit des DVT Systems aus Abb. 1, wobei der Behandler unterschiedliche vordefinierte Volumengrößen auswählen kann,
Abb.2c - zeigt ein Zahnschema in dem der Behandler den Zahn, für den er hochauflösende Informationen haben möchte markiert;
Abb.2d - zeigt die Bedienungseinheit des DVT Systems aus Abb. 1, wobei der Behandler eine Region auf der Bedienoberfläche bestimmt für die er hochauflösende Informationen haben möchtet;
Abb.3 - zeigt eine schematische 3D Scoutaufnahme nach einer Ausführungsform in der der Behandler den Zahn, für den er hochauflösende Informationen haben möchte, mit einer umhüllenden Geometrie markiert hat;
Abb.4 - zeigt die Abbildungspositionen der umhüllenden Geometrie auf dem Röntgendetektor für zwei verfügbaren Drehzentren jeweils in der oberen/unteren Reihen, bei denen die umhüllenden Geometrie abgebildet werden kann nach einer Ausführungsform,
Abb.5 - zeigt den zeitlichen Verlauf von acht Abbildungspositionen der umhüllenden Geometrie in der X-Richtung und Y-Richtung des Röntgendetektors für das verfügbare Drehzentrum entsprechend der unteren Reihe aus Abb. 4.

Die in den Zeichnungen gezeigten Referenznummern bezeichnen die unten aufgeführten Elemente, auf die in der nachfolgenden Beschreibung der beispielhaften Ausführungsformen Bezug genommen wird.
- 1.: DVT-System
- 2.: Röntgengerät
- 3.: Röntgenstrahler
- 4.: Röntgendetektor
- 4a.: Detektorbereiche (gestrichelt)
- 5.: Bedienungseinheit /Bedieneroberfläche
- 5a.: Zahnschema
- 5b.: Region
- 6.: Kopffixierung / Schläfenstützen
- 7.: Aufbiss
- 8.: Rechner
- 9.: Anzeige
- 9a.: 3D Scoutaufnahme
- 9b.: Hochauflösende Aufnahme
- 10.: Verfügbare Volumengröße
- 10': Vordefinierte Volumengröße
- 11.: Verfügbare Drehzentren
- 11': Vordefiniertes Drehzentrum
- 11": Ausgewähltes Drehzentrum
- 12.: Zahn
- 12a.: Kieferbogen
- 13: Umhüllende Geometrie
- 13a-13h: Referenzpunkten (z.B. Ecken)
- 13': Umhüllende Geometrie (abgebildet)
- 13a'-13h': Abbildungspositionen
- 14a-14d: Blendenpositionen

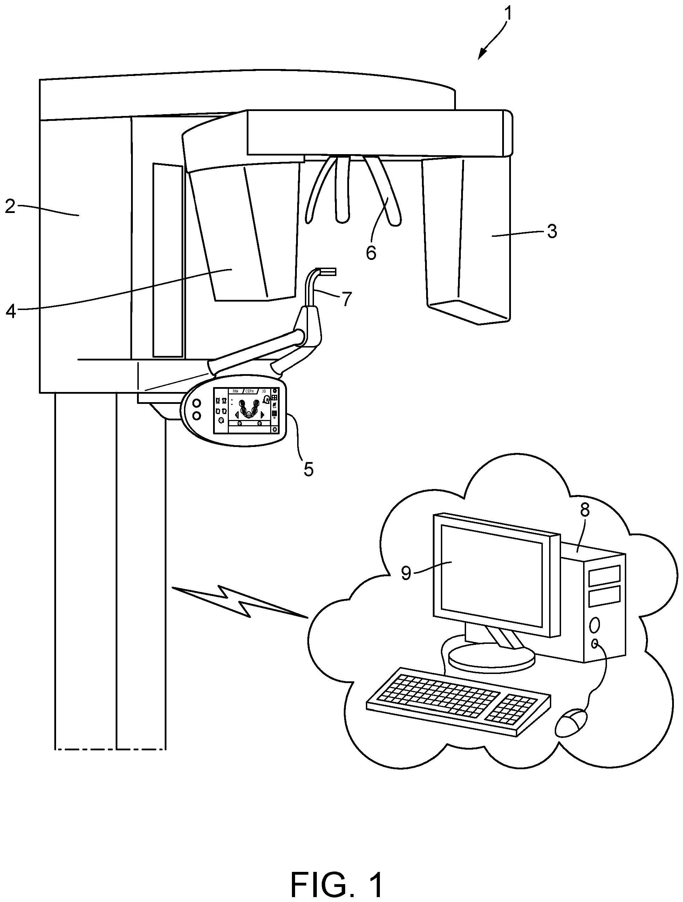

Die alternative erfindungsgemäßen Verfahren sind Computer implementierte Verfahren, und können jeweils auf einem computergestützten DVT-System (1) ausgeführt werden. Die Verfahren dienen zur digitalen Volumen Tomographie (DVT) Aufnahme im dentalen Bereich. Abb. 1 zeigt eine Ausführungsform des DVT-Systems (1).

Die erfindungsgemäßen Verfahren werden durch Computerprogramme implementiert, welche computerlesbare Code aufweisen. Jedes Computerprogramm kann auf einem Datenspeicher bereitgestellt werden.

Wie in Abb. 1 gezeigt umfasst das computergestützte DVT-System (1) ein Röntgengerät (2) zur Durchführung der Patientenaufnahme, womit die einzelnen 2D Röntgenbilder bzw. ein Sinogramm erzeugt wird. Vor der Aufnahme wird der Patientenkopf mit dem Aufbiss (7) und der Kopffixierung (6) im Röntgengerät (2) positioniert. Das Röntgengerät (2) hat einen Röntgenstrahler (3) und Röntgendetektor (4), die während der Aufnahme um den Patiententkopf gedreht werden.

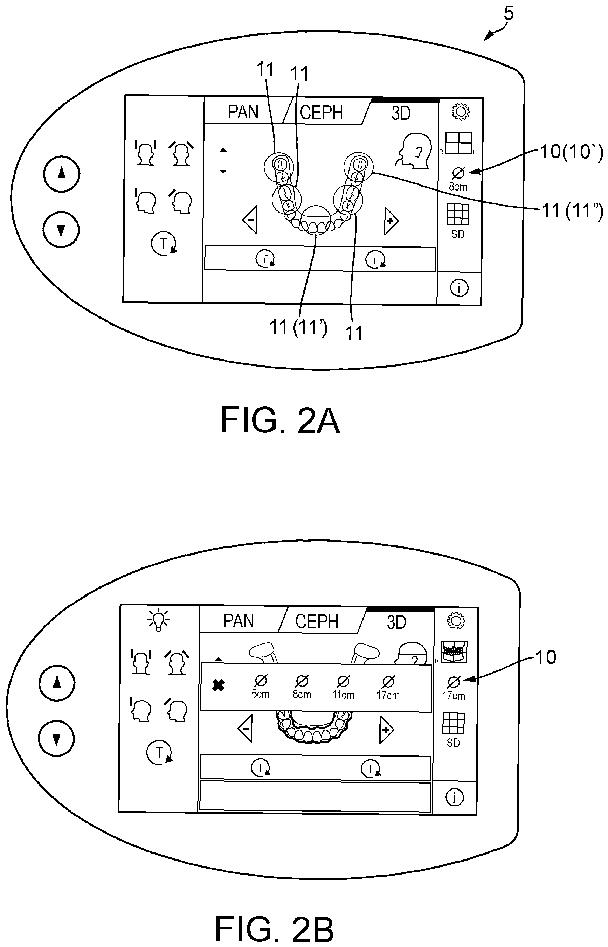

Wie in Abb. 1 gezeigt hat das computergestützte DVT-System (1) eine Bedienungseinheit (5). Abb. 2A zeigt eine vergrößerte Detail Ansicht eines Dialogfelds auf der Bedienungseinheit (5), in dem die verfügbaren (kalibrierten) Drehzentren (11) gezeigt werden. Das Röntgengerät (2) hat einen Drehmechanismus (nicht gezeigt) wodurch der Röntgenstrahler (3) und der Röntgendetektor (4) um den Patiententkopf gemäß einer der verfügbaren Drehzentren (11) wahlweise gedreht werden kann. Der Drehmechanismus kann für jedes verfügbare Drehzentrum (11) eine entsprechend vordefinierte Bahnkurve fahren, welche aus einer vordefinierten Röntgenstrahler-Trajektorie und einer vordefinierten Röntgendetektor-Trajektorie besteht. Die Bahnkurven können jeweils eine Kreisbahn beschreiben. Alternativ können sie vorzugsweise eine davon abweichende komplexere Kurvenform annehmen, die den Platzbedarf reduziert und dabei die Schulterfreiheit berücksichtigt. Abb. 2B zeigt eine vergrößerte Detail Ansicht eines Dialogfelds auf der Bedienungseinheit (5), wobei der Behandler unterschiedliche verfügbare Volumengrößen (10) auswählen kann mit z.B. 5, 8, 11, und 17 cm Durchmesser.

Wie in Abb. 1 gezeigt, hat das computergestützte DVT-System (1) einen separaten Rechner (8) oder eine Recheneinheit, die mit dem Röntgengerät (2) verbunden werden kann, und eine separate Anzeige (9), u.a. um Datensätze zu visualisieren. Der Rechner (8) kann über ein lokales Netzwerk (nicht gezeigt) oder alternativ über das Internet mit dem Röntgengerät (2) verbunden werden. Der Rechner (8) kann Teil einer Cloud sein. Alternativ kann der Rechner (8) in das Röntgengerät (2) integriert werden. Die Berechnungen können im Rechner (8) bzw. in der Cloud stattfinden. Der Rechner (8) führt das Computerprogramm aus und liefert die Datensätze, u.a. auch für die Visualisierung auf der Anzeige (9). Die Anzeige (9) kann räumlich von dem Röntgengerät (2) getrennt sein. Der Rechner (8) kann vorzugsweise auch das Röntgengerät (2) steuern. Alternativ können separate Rechner (8) für die Steuerung und die Bildbearbeitung benutzt werden.

In der folgenden Beschreibung werden die Verfahren gemäß alternativer Ausführungsformen naher erklärt.

### 1. Ausführungsform

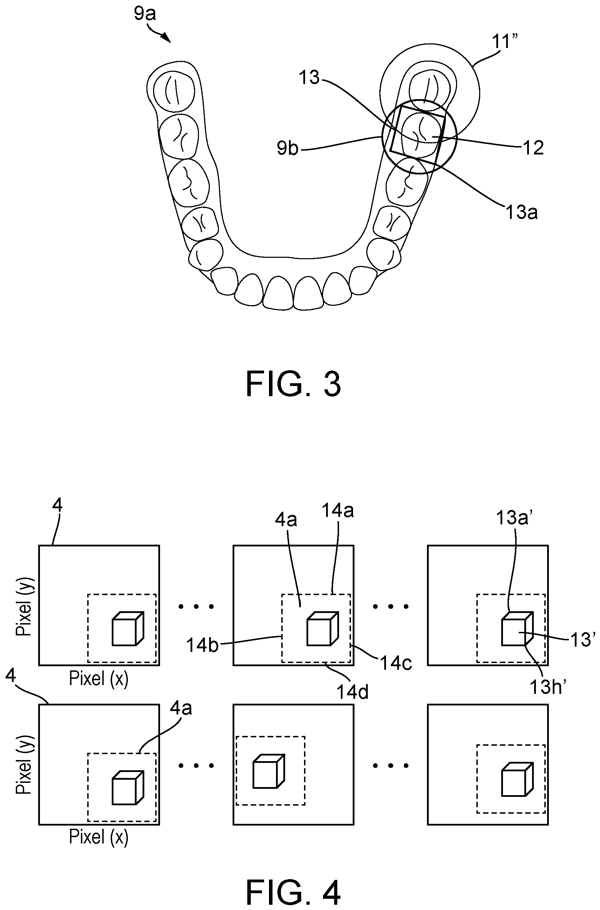

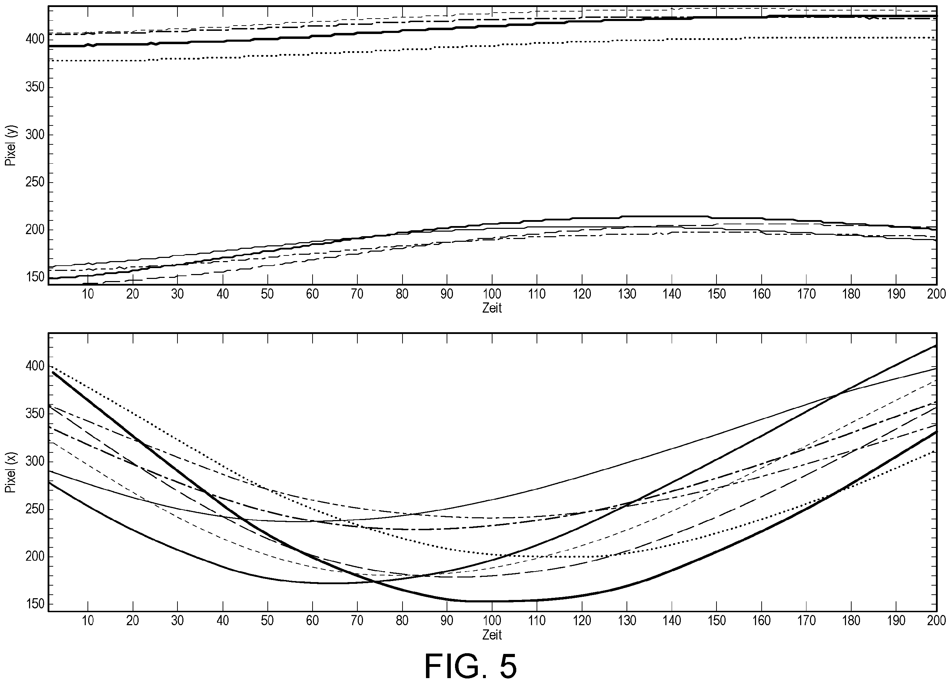

Das Verfahren nach der ersten Ausführungsform umfasst die Schritte (a) bis (h). Im Schritt (a) wird der Patient im Röntgengerät (2) positioniert (nicht gezeigt). Im Schritt (b) wird eine 3D Scoutaufnahme (9a) mit reduzierter Dosis und mit einer der vordefinierten Volumengrößen (10') und einem vordefinierten Drehzentrum (11') erstellt. Fig. 3 zeigt einen schematische axiale Querschnittsdarstellung der 3D Scoutaufnahme (9a). Fig. 2A zeigt die vordefinierte Volumengröße (10') und das vordefinierte Drehzentrum (11'). Wobei die vordefinierte Volumengröße (10') beispielsweise 8cm Durchmesser hat und das vordefinierte Drehzentrum (11') beispielsweise mittig an der vorderen Seite des Kieferbogens liegt. Im Schritt (c) markiert der Behandler auf der 3D Scoutaufnahme (9a) wie in Fig. 3 gezeigt den Zahn (12), für den er hochauflösende Informationen (9b) haben möchte, mit einer umhüllenden Geometrie (13). Die umhüllende Geometrie (13) hat beispielsweise eine Quaderform mit 8 Ecken, die als Referenzpunkte (13a-13h) dienen werden. Im Schritt (d) bestimmt eine Software anhand der Position der umhüllenden Geometrie (13) die verfügbaren Drehzentren (11), bei denen diese umhüllenden Geometrie (13) abgebildet werden kann, und errechnet anhand von den Referenzpunkten (13a-13h) der umhüllenden Geometrie (13) wie in Abb.4 gezeigt die Abbildungspositionen (13a'-13h') auf dem Röntgendetektor (4) für jedes der verfügbaren Drehzentren (11). In Abb.4 entsprechen die oberen und unteren Reihen des Röntgendetektor (4) jeweils zwei verfügbaren Drehzentren (11) bei denen die umhüllende Geometrie (13') beispielsweise abgebildet werden kann. Abb. 5 zeigt den zeitlichen Verlauf von acht Abbildungspositionen (13a'-13h') der umhüllenden Geometrie (13) entlang der Y-Richtung (obere Graphik) und entlang der X-Richtung (untere Graphik) des Röntgendetektors (4) für eines der verfügbaren Drehzentren entsprechend der unteren Reihe aus Abb. 4. Im Schritt (e) wird anhand der Abbildungspositionen (13a' -13h') ein Drehzentrum (11") aus der Menge der verfügbaren Drehzentren (11) ausgewählt, und zu diesem Drehzentrum (11") werden die Blendenpositionen (14a-d) für die Aufnahme und die zu bestrahlende Detektorbereiche (4a) der Aufnahme ermittelt. In diesem Beispiel können vier Blendenpositionen (14a-d) separat ermittelt werden, welche die abgebildete umhüllenden Geometrie (13') von oben, links, unten und rechts einrahmen (s. gestricheltes Viereck). Im Schritt (f) werden die zuvor ermittelnden Blendenpositionen (14a-d) und die zu bestrahlenden Detektorbereiche (4a) an das Röntgengerät (2) übermittelt und nun wird die hochauflösende Aufnahme (9b) mit dem aus der Menge der Verfügbaren, ausgewählten Drehzentrum (11") und den übermittelten Blendenpositionen (14a-d) durchgeführt. Im Schritt (g) wird die hochauflösende Aufnahme (9b) rekonstruiert und in der 3D Scoutaufnahme (9a) die neu aufgenommene hochauflösende Information (9b) eingeblendet. Im Schritt (h) erhält der Behandler zur Befundung und weiteren Verwendung die 3D Scoutaufnahme (9a) wie in Abb. 3 schematisch dargestellt, bei der die zuvor markierte umhüllende Geometrie (13) durch die hochauflösende Information (9b) überlagert ist.

### 2. Ausführungsform

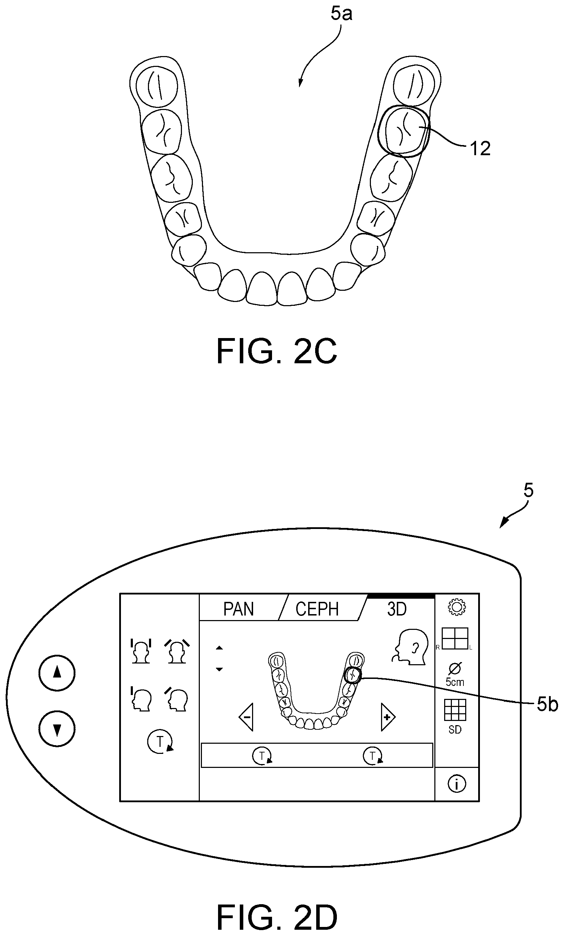

Das Verfahren nach der zweiten Ausführungsform umfasst die Schritte (a) bis (h), wie in der ersten 1. Ausführungsform, wobei die Schritte (b) und (c) durch die Schritte (b1'), (b2'), (b3') und (c') ersetzt werden. Im Schritt (b1') markiert der Behandler wie in Abb. 2c gezeigt, den Zahn (12), für den er hochauflösende Informationen (9b) haben möchte anhand eines Zahnschemas (5a). Das Zahnschema (5a) kann auf der Anzeige (9) bzw. auf dem Display der Bedienungseinheit/Bedieneroberfläche (5) gezeigt werden. Das Markieren kann mit der Maus, Tastatur oder ein Lichtstift erfolgen. Die Displays können touch-sensitive sein. Im Schritt (b2') bestimmt das Röntgengerät (2) automatisiert das optimale verfügbare kalibrierte Drehzentrum (11') für die 3D Scoutaufnahme (9a), das diesen markierten Zahn (12) sicher abbildet. Im Schritt (b3') wird eine 3D Scoutaufnahme (9a) mit reduzierter Dosis und mit einer der vordefinierten Volumengrößen (10) und dem optimalen verfügbaren kalibrierten Drehzentrum (11') aus dem vorangegangenen Schritt erstellt. Im Schritt (c') wird mittels einer Software der markierte Zahn (12) auf der 3D Scoutaufnahme gefunden (9a) und eine, die den markierten Zahn (12) umhüllende Geometrie (13) automatisch bestimmt. Der weitere Verlauf des Verfahrens ist wie in den Schritten (d) bis (h) wie in der 1. Ausführungsform. Diese werden hier nicht unnötig wiederholt.

### 3. Ausführungsform

Das Verfahren nach der dritten Ausführungsform umfasst die Schritte (a) bis (g), wie in der ersten Ausführungsform, wobei die Schritte (b) und (c) durch die Schritte (b"), (c") ersetzt und wobei der Schritt (g) durch den Schritt (g') ersetzt wird, und der Schritt (h) wegfällt. Im Schritt (b") bestimmt der Behandler eine Region (5b), wie in Abb. 2d gezeigt, auf der Bedienoberfläche (5) für die er hochauflösende Informationen (9b) haben möchte. Im Schritt (c") bestimmt eine Software anhand der gewählten Region (5b) und der Position der geschlossenen Schläfenstützen (6) oder eines anderen Hilfsmittels eine umhüllende Geometrie (13). Der weitere Verlauf des Verfahrens ist wie in den Schritten (d) bis (f) der 1. Ausführungsform. Diese werden hier nicht unnötig wiederholt. Im Schritt (g') wird die hochauflösende Aufnahme (9b) rekonstruiert und dem Behandler zur Befundung dargestellt.

Gemäß der vorliegenden Erfindung können die Datensätze, die durch die oben aufgeführte Ausführungsformen erzeugt werden, zur Visualisierung, insbesondere für diagnostische Zwecke, einem Arzt vorgelegt werden, vorzugsweise mittels der Anzeige (9) oder eines Ausdrucks.

In weiteren bevorzugten Varianten der oben beschriebenen Verfahren wird im Schritt (e) anhand der Abbildungspositionen (13a' -13h') das Drehzentrum (11") ausgewählt, dass die *vorteilhafteste* Abbildungsposition (13a'-13h') auf dem Röntgendetektor (4) bietet. Bei der Ermittlung der vorteilhaftesten Abbildungsposition (13a'-13h') werden Einflüsse wie, die Abbildungsverzerrung durch den Anodenwinkel berücksichtigt. Alternativ oder zusätzlich wird bei der Ermittlung der vorteilhaftesten Abbildungsposition (13a'-13h') eines oder mehrerer der Kriterien wie Patient-Detektor-Abstand, Minimierung der Effektivdosis, Minimierung der Blendenbewegung, Minimierung der Anzahl der Blendenpositionen (14a-d), oder Minimierung der Größe der zu bestrahlenden Detektorbereiche (4a) berücksichtigt.

## Patentansprüche

1. Verfahren zur digitalen Volumen Tomographie (DVT) Aufnahme im dentalen Bereich mit einem Röntgengerät (2), welches die folgenden Schritte umfasst:
(a) Ein Patient wird im Röntgengerät (2) positioniert;
(b) Es wird eine 3D Scoutaufnahme des Patienten (9a) mit reduzierter Dosis und mit einer von mehreren vordefinierten Volumengrößen (10) und einem vordefinierten Drehzentrum (11') des Röntgengeräts (2) erstellt:
(c) Auf der 3D Scoutaufnahme (9a) markiert ein Behandler einen Zahn (12), für den er hochauflösende Informationen (9b) haben möchte, mit einer umhüllenden Geometrie (13);
(d) Eine Software bestimmt anhand einer Position der umhüllenden Geometrie (13) die verfügbaren Drehzentren (11) des Röntgengeräts (2), bei denen dieser umhüllenden Geometrie (13) abgebildet werden kann, und errechnet anhand von Referenzpunkten (13a-13h) der umhüllenden Geometrie (13) die Abbildungspositionen (13a'-13h') auf einem Röntgendetektor (4) des Röntgengeräts (2) für jedes der verfügbaren Drehzentren (11);
(e) Anhand der Abbildungspositionen (13a' -13h') wird ein Drehzentrum (11") aus der Menge der verfügbaren Drehzentren (11) ausgewählt, und zu diesem Drehzentrum (11") werden Blendenpositionen (14a-d) für eine hochauflösende Aufnahme und zu bestrahlende Detektorbereiche (4a) der hochauflösenden Aufnahme ermittelt;
(f) Die zuvor ermittelnden Blendenpositionen (14a-d) und die zu bestrahlenden Detektorbereiche (4a) werden an das Röntgengerät (2) übermittelt und nun die hochauflösende Aufnahme mit dem aus der Menge der verfügbaren Drehzentren (11) ausgewählten Drehzentrum (11") und den übermittelten Blendenpositionen (14a-d) durchgeführt;
(g) Die hochauflösende Aufnahme (9b) wird rekonstruiert und in der 3D Scoutaufnahme (9a) eine entsprechende neu aufgenommene hochauflösende Information (9b) eingeblendet;
(h) Der Behandler erhält zur Befundung und weiteren Verwendung die 3D Scoutaufnahme (9a) dargestellt, bei der die zuvor markierte umhüllende Geometrie (13) durch die hochauflösende Information (9b) überlagert ist.

2. Verfahren zur digitalen Volumen Tomographie (DVT) Aufnahme im dentalen Bereich mit einem Röntgengerät (2), welches die folgenden Schritte umfasst:
(a) Ein Patient wird im Röntgengerät (2) positioniert;
(b1') Ein Behandler markiert einen Zahn (12), für den er hochauflösende Informationen haben möchte anhand eines Zahnschemas (5a);
(b2') Das Röntgengerät (2) bestimmt automatisiert ein optimales kalibriertes Drehzentrum (11') des Röntgengeräts (2) für die 3D Scoutaufnahme (9a), das diesen markierten Zahn (12) sicher abbildet;
(b3') Es wird eine 3D Scoutaufnahme (9a) mit reduzierter Dosis und mit einer von mehreren vordefinierten Volumengrößen (10) und dem optimalen kalibrierten Drehzentrum (11') erstellt;
(c') mittels einer Software wird der markierte Zahn (12) auf der 3D Scoutaufnahme gefunden (9a) und eine die den markierten Zahn (12) umhüllende Geometrie (13) automatisch bestimmt.
(d) Eine Software bestimmt anhand einer Position der umhüllenden Geometrie (13) die verfügbaren Drehzentren (11) des Röntgengeräts (2), (11), bei denen diese umhüllende Geometrie (13) abgebildet werden kann, und errechnet anhand von Referenzpunkten (13a-13h) der umhüllenden Geometrie (13) die Abbildungspositionen (13a'-13h') auf einem Röntgendetektor (4) des Röntgengeräts (2) für jedes der verfügbaren Drehzentren (11);
(e) Anhand der Abbildungspositionen (13a' -13h') wird ein Drehzentrum (11") aus der Menge der verfügbaren Drehzentren (11) ausgewählt, und zu diesem Drehzentrum (11") werden Blendenpositionen (14a-d) für eine hochauflösende Aufnahme und zu bestrahlende Detektorbereiche (4a) für die hochauflösende Aufnahme ermittelt;
(f) Die zuvor ermittelnden Blendenpositionen (14a-d) und zu bestrahlenden Detektorbereiche (4a) werden an das Röntgengerät (2) übermittelt und nun die hochauflösende Aufnahme mit dem aus der Menge der verfügbaren Drehzentren (11) ausgewählten Drehzentrum (11") und den übermittelten Blendenpositionen (14a-d) durchgeführt;
(g) Die hochauflösende Aufnahme (9b) wird rekonstruiert und in der 3D Scoutaufnahme (9a) eine entsprechende neu aufgenommene hochauflösende Information (9b) eingeblendet;
(h) Der Behandler erhält zur Befundung und weiteren Verwendung die 3D Scoutaufnahme (9a) dargestellt, bei der die zuvor markierte umhüllende Geometrie (13) durch die hochauflösende Information (9b) überlagert ist.

3. Verfahren zur digitalen Volumen Tomographie (DVT) Aufnahme im dentalen Bereich mit einem Röntgengerät (2), welches die folgenden Schritte umfasst:
(a) Ein Patient wird im Röntgengerät (2) positioniert;
(b") Ein Behandler bestimmt eine Region (5b) auf einer Bedienoberfläche (5) für die er hochauflösende Informationen (9b) haben möchte;
(c") Eine Software bestimmt Anhand der gewählten Region (5b) und einer Position von geschlossenen Schläfenstützen (6) oder eines anderen Hilfsmittels eine umhüllende Geometrie (13);
(d) Eine Software bestimmt anhand einer Position der umhüllenden Geometrie (13) die verfügbaren Drehzentren (11) des Röntgengeräts (2), bei denen diese Hülle (13) abgebildet werden kann und errechnet anhand von Referenzpunkten (13a-13h) der umhüllenden Geometrie (13) entsprechende Abbildungspositionen (13a'-13h') auf einem Röntgendetektor (4) des Röntgengeräts (2) für jedes der verfügbaren Drehzentren (11);
(e) Anhand der Abbildungspositionen (13a'-13h') wird ein Drehzentrum (11") aus der Menge der verfügbaren Drehzentren (11) ausgewählt, und zu diesem Drehzentrum (11") werden Blendenpositionen (14a-d) für eine hochauflösende Aufnahme und zu bestrahlende Detektorbereiche (4a) für die hochauflösende Aufnahme ermittelt;
(f) Die zuvor ermittelnden Blendenpositionen (14a-d) und zu bestrahlenden Detektorbereiche (4a) werden an das Röntgengerät (2) übermittelt und nun die hochauflösende Aufnahme mit dem aus der Menge der verfügbaren Drehzentren (11) ausgewählten Drehzentrum (11") und den übermittelten Blendenpositionen (14a-d) durchgeführt;
(g') Die hochauflösende Aufnahme (9b) wird rekonstruiert und dem Behandler zur Befundung dargestellt.

4. Verfahren nach einem der vorherigen Ansprüche, wobei im Schritt (e) anhand der Abbildungspositionen (13a'-13h') das Drehzentrum (11") ausgewählt wird, das die vorteilhafteste Abbildungsposition (13a'-13h') auf dem Röntgendetektor (4) bietet, unter Berücksichtigung von Einflüssen wie der Abbildungsverzerrung durch den Anodenwinkel, oder Kriterien wie Patient-Detektor-Abstand, oder Minimierung der Effektivdosis, oder Minimierung der Blendenbewegung, oder Minimierung der Anzahl der Blendenpositionen (14a-d), oder Minimierung der Größe der zu bestrahlenden Detektorbereiche (4a).

5. Computerprogramm umfassend computerlesbaren Code, welcher, wenn er von einem computergestützten DVT-System (1) ausgeführt wird, dieses veranlasst, die Verfahrensschritte einer der vorhergehenden Verfahrensansprüche auszuführen.

6. Computergestütztes DVT-System (1) umfassend ein Röntgengerät (2) und eine Recheneinheit (8) die zur Ausführung des Computerprogramms nach Anspruch 5 konfiguriert ist.

## Claims

1. A method for digital volume tomography (DVT) imaging in the dental region with an X-ray apparatus (2), comprising the following steps:
(a) A patient is positioned in the X-ray apparatus (2);
(b) A 3D scout image of the patient (9a) is taken with reduced dose and with one of a plurality of predefined volume sizes (10) and a predefined center of rotation (11') of the X-ray apparatus (2);
(c) A practitioner marks a tooth (12) on the 3D scout image (9a) for which he or she wants high-resolution information (9b) with an enveloping geometry (13);
(d) A software uses a position of the enveloping geometry (13) to determine the available centers of rotation (11) of the X-ray apparatus (2) at which this enveloping geometry (13) can be imaged, and uses reference points (13a-13h) of the enveloping geometry (13) to calculate the imaging positions (13a'-13h') on an X-ray detector (4) of the X-ray apparatus (2) for each of the available centers of rotation (11);
(e) Based on the imaging positions (13a'-13h'), a center of rotation (11") is selected from the set of available centers of rotation (11), and aperture positions (14a-d) for a high-resolution image and detector areas (4a) of the high-resolution image to be irradiated are determined for this center of rotation (11");
(f) The previously determined aperture positions (14a-d) and the detector areas (4a) to be irradiated are transmitted to the X-ray apparatus (2) and the high-resolution exposure is now performed with the center of rotation (11") selected from the set of available centers of rotation (11) and the transmitted aperture positions (14a-d);
(g) The high-resolution image (9b) is reconstructed and a corresponding newly recorded high-resolution information (9b) is superimposed on the 3D scout image (9a);
(h) The 3D scout image (9a), in which the previously marked enveloping geometry (13) is superimposed by the high-resolution information (9b), is displayed to the practitioner for diagnosis and further use.

2. A method for digital volume tomography (DVT) imaging in the dental region with an X-ray apparatus (2), comprising the following steps:
(a) A patient is positioned in the X-ray apparatus (2);
(b1') A practitioner marks a tooth (12) for which he wishes to have high-resolution information using a tooth scheme (5a);
(b2') The X-ray apparatus (2) automatically determines an optimal calibrated center of rotation (11') of the X-ray apparatus (2) for the 3D scout image (9a), which reliably images this marked tooth (12);
(b3') A 3D scout image (9a) is created with reduced dose and with one of a plurality of predefined volume sizes (10) and the optimum calibrated center of rotation (11');
(c') The marked tooth (12) is found on the 3D scout image (9a) by means of software and a geometry (13) enveloping the marked tooth (12) is automatically determined.
(d) A software uses a position of the enveloping geometry (13) to determine the available centers of rotation (11) of the X-ray apparatus (2) at which this enveloping geometry (13) can be imaged, and uses reference points (13a-13h) of the enveloping geometry (13) to calculate the imaging positions (13a'-13h') on an X-ray detector (4) of the X-ray apparatus (2) for each of the available centers of rotation (11);
(e) Based on the imaging positions (13a'-13h'), a center of rotation (11") is selected from the set of available centers of rotation (11), and aperture positions (14a-d) for a high-resolution image and detector areas (4a) for the high-resolution image to be irradiated are determined for this center of rotation (11");
(f) The previously determined aperture positions (14a-d) and the detector areas (4a) to be irradiated are transmitted to the X-ray apparatus (2) and the high-resolution exposure is now performed with the center of rotation (11") selected from the set of available centers of rotation (11) and the transmitted aperture positions (14a-d);
(g) The high-resolution image (9b) is reconstructed and a corresponding newly recorded high-resolution information (9b) is superimposed on the 3D scout image (9a);
(h) The 3D scout image (9a), in which the previously marked enveloping geometry (13) is superimposed by the high-resolution information (9b), is displayed to the practitioner for diagnosis and further use.

3. A method for digital volume tomography (DVT) imaging in the dental region with an X-ray apparatus (2), comprising the following steps:
(a) A patient is positioned in the X-ray apparatus (2);
(b") A practitioner specifies a region (5b) on a user interface (5) for which he wishes to have high-resolution information (9b);
(c") A software determines an enveloping geometry (13) based on the selected region (5b) and a position of closed temple supports (6) or another aid;
(d) A software uses a position of the enveloping geometry (13) to determine the available centers of rotation (11) of the X-ray apparatus (2) at which this envelope (13) can be imaged, and uses reference points (13a-13h) of the enveloping geometry (13) to calculate the corresponding imaging positions (13a'-13h') on an X-ray detector (4) of the X-ray apparatus (2) for each of the available centers of rotation (11);
(e) Based on the imaging positions (13a'-13h'), a center of rotation (11") is selected from the set of available centers of rotation (11), and aperture positions (14a-d) for a high-resolution image and detector areas (4a) for the high-resolution image to be irradiated are determined for this center of rotation (11");
(f) The previously determined aperture positions (14a-d) and the detector areas (4a) to be irradiated are transmitted to the X-ray apparatus (2) and the high-resolution exposure is now performed with the center of rotation (11") selected from the set of available centers of rotation (11) and the transmitted aperture positions (14a-d);
(g') The high-resolution image (9b) is reconstructed and presented to the practitioner for diagnosis.

4. The method according to any one of the preceding claims, wherein in step (e) the center of rotation (11") providing the most advantageous imaging position (13a'-13h') on the X-ray detector (4) is selected on the basis of the imaging positions (13a'-13h'), taking into account influences such as the imaging distortion due to the anode angle, or criteria such as patient-detector distance, or minimization of the effective dose, or minimization of the aperture movement, or minimization of the number of aperture positions (14a-d), or minimization of the size of the detector areas (4a) to be irradiated.

5. A computer program comprising computer readable code which, when executed by a computer-assisted DVT system (1), causes it to perform the method steps of any of the preceding method claims.

6. A computer-assisted DVT system (1) comprising an X-ray apparatus (2) and a computing unit (8) configured to execute the computer program according to claim 5.

## Revendications

1. Procédé de prise de vue par tomographie volumique numérique (TVN) dans le domaine dentaire à l'aide d'un appareil radiographique (2), comprenant les étapes suivantes :
(a) Un patient est positionné dans l'appareil radiographique (2) ;
(b) Une prise de vue 3D Scout du patient (9a) est réalisée avec une dose réduite et avec une taille de volume (10) prédéfinie parmi plusieurs et un centre de rotation (11') prédéfini de l'appareil radiographique (2) ;
(c) Sur la prise de vue 3D Scout (9a), un praticien marque une dent (12), pour laquelle il souhaite obtenir des informations à haute résolution (9b), à l'aide d'une géométrie enveloppante (13) ;
(d) Un logiciel détermine, à partir d'une position de la géométrie enveloppante (13), les centres de rotation (11) disponibles de l'appareil radiographique (2) pour lesquels cette géométrie enveloppante (13) peut être reproduite, et calcule, à partir de points de référence (13a-13h) de la géométrie enveloppante (13), les positions d'imagerie (13a'-13h') sur un détecteur de rayons X (4) de l'appareil radiographique (2) pour chacun des centres de rotation (11) disponibles ;
(e) À l'aide des positions d'imagerie (13a'-13h'), un centre de rotation (11") est sélectionné parmi l'ensemble des centres de rotation (11) disponibles, et pour ce centre de rotation (11"), des positions de diaphragme (14a-d) sont déterminées pour une prise de vue à haute résolution et des zones de détection (4a) à irradier de la prise de vue à haute résolution ;
(f) Les positions de diaphragme (14a-d) précédemment déterminées et les zones de détection (4a) à irradier sont transmises à l'appareil radiographique (2) et la prise de vue à haute résolution est alors effectuée avec le centre de rotation (11") sélectionné parmi l'ensemble des centres de rotation (11) disponibles et avec les positions de diaphragme (14a-d) transmises ;
(g) La prise de vue à haute résolution (9b) est reconstruite et une information à haute résolution (9b) correspondante nouvellement prise est insérée dans la prise de vue 3D Scout (9a)
(h) Le praticien reçoit, pour analyse et pour une utilisation ultérieure, la prise de vue 3D Scout (9a) représentée, dans laquelle la géométrie enveloppante (13) précédemment marquée est superposée par l'information à haute résolution (9b).

2. Procédé de prise de vue par tomographie volumique numérique (TVN) dans le domaine dentaire à l'aide d'un appareil radiographique (2), comprenant les étapes suivantes :
(a) Un patient est positionné dans l'appareil radiographique (2) ;
(b1') Un praticien marque une dent (12) pour laquelle il souhaite obtenir des informations à haute résolution à l'aide d'un schéma dentaire (5a) ;
(b2') L'appareil radiographique (2) détermine de manière automatisée un centre de rotation (11') calibré optimal de l'appareil radiographique (2) pour la prise de vue 3D Scout (9a), qui reproduit de manière sûre cette dent marquée (12) ;
(b3') Une prise de vue 3D Scout (9a) est réalisée avec une dose réduite et avec une taille de volume (10) prédéfinie parmi plusieurs et le centre de rotation (11') calibré optimal ;
(c') Au moyen d'un logiciel, la dent marquée (12) est trouvée sur la prise de vue 3D Scout (9a) et une géométrie (13) enveloppant la dent marquée (12) est déterminée automatiquement.
(d) Un logiciel détermine, à partir d'une position de la géométrie enveloppante (13), les centres de rotation (11) disponibles de l'appareil radiographique (2) pour lesquels cette géométrie enveloppante (13) peut être reproduite, et calcule, à partir de points de référence (13a-13h) de la géométrie enveloppante (13), les positions d'imagerie (13a'-13h') sur un détecteur de rayons X (4) de l'appareil radiographique (2) pour chacun des centres de rotation (11) disponibles ;
(e) À l'aide des positions d'imagerie (13a'-13h'), un centre de rotation (11") est sélectionné parmi l'ensemble des centres de rotation (11) disponibles, et pour ce centre de rotation (11"), des positions de diaphragme (14a-d) sont déterminées pour une prise de vue à haute résolution et des zones de détection (4a) à irradier pour la prise de vue à haute résolution ;
(f) Les positions de diaphragme (14a-d) précédemment déterminées et les zones de détection (4a) à irradier sont transmises à l'appareil radiographique (2) et la prise de vue à haute résolution est alors effectuée avec le centre de rotation (11") sélectionné parmi l'ensemble des centres de rotation (11) disponibles et avec les positions de diaphragme (14a-d) transmises ;
(g) La prise de vue à haute résolution (9b) est reconstruite et une information à haute résolution (9b) correspondante nouvellement prise est insérée dans la prise de vue 3D Scout (9a)
(h) Le praticien reçoit, pour analyse et pour une utilisation ultérieure, la prise de vue 3D Scout (9a) représentée, dans laquelle la géométrie enveloppante (13) précédemment marquée est superposée par l'information à haute résolution (9b).

3. Procédé de prise de vue par tomographie volumique numérique (TVN) dans le domaine dentaire à l'aide d'un appareil radiographique (2), comprenant les étapes suivantes :
(a) Un patient est positionné dans l'appareil radiographique (2) ;
(b") Un praticien détermine une région (5b) sur une interface utilisateur (5) pour laquelle il souhaite obtenir des informations à haute résolution (9b) ;
(c") Un logiciel détermine une géométrie enveloppante (13) à l'aide de la région sélectionnée (5b) et d'une position de supports temporaux fermés (6) ou d'un autre moyen auxiliaire ;
(d) Un logiciel détermine, à partir d'une position de la géométrie enveloppante (13), les centres de rotation (11) disponibles de l'appareil radiographique (2) pour lesquels cette enveloppe (13) peut être reproduite, et calcule, à partir de points de référence (13a-13h) de la géométrie enveloppante (13), des positions d'imagerie (13a'-13h') correspondantes sur un détecteur de rayons X (4) de l'appareil radiographique (2) pour chacun des centres de rotation (11) disponibles
(e) À l'aide des positions d'imagerie (13a'-13h'), un centre de rotation (11") est sélectionné parmi l'ensemble des centres de rotation (11) disponibles, et pour ce centre de rotation (11"), des positions de diaphragme (14a-d) sont déterminées pour une prise de vue à haute résolution et des zones de détection (4a) à irradier pour la prise de vue à haute résolution ;
(f) Les positions de diaphragme (14a-d) précédemment déterminées et les zones de détection (4a) à irradier sont transmises à l'appareil radiographique (2) et la prise de vue à haute résolution est alors effectuée avec le centre de rotation (11") sélectionné parmi l'ensemble des centres de rotation (11) disponibles et avec les positions de diaphragme (14a-d) transmises ;
(g') La prise de vue à haute résolution (9b) est reconstruite et présentée au praticien pour analyse.

4. Procédé selon l'une des revendications précédentes, où, à l'étape (e), à l'aide des positions d'imagerie (13a'-13h'), on sélectionne le centre de rotation (11") qui offre la position d'imagerie (13a'-13h') la plus avantageuse sur le détecteur de rayons X (4), en tenant compte d'influences telles que la distorsion d'image due à l'angle de l'anode, ou des critères tels que la distance patient-détecteur, ou la minimisation de la dose efficace, ou la minimisation du mouvement du diaphragme, ou la minimisation du nombre de positions du diaphragme (14a-d), ou la minimisation de la taille des zones de détection (4a) à irradier.

5. Programme informatique comprenant un code lisible par ordinateur qui, lorsqu'il est exécuté par un système de TVN assisté par ordinateur (1), amène ce dernier à exécuter les étapes de procédé selon l'une des revendications de procédé précédentes.

6. Système de TVN assisté par ordinateur (1) comprenant un appareil radiographique (2) et une unité de calcul (8) configurée pour exécuter le programme informatique selon la revendication 5.
